# EUROPEAN PATENT APPLICATION

(11) **EP 4 129 050 A1**
(43) Date of publication of application: **08.02.2023**
(21) Application number: 21776678.1
(22) Date of filing: 25.03.2021
(51) Int. Cl.: A01H 1/00, A01H 1/02, A01H 5/10, C12N 15/29

(54) **METHOD FOR PRODUCING TEMPERATURE-SENSITIVE MALE STERILE PLANT**

(30) Priority: 26.03.2020 JP 2020056381
(71) Applicant: NATIONAL AGRICULTURE AND FOOD RESEARCH ORGANIZATION, Tsukuba-shi, Ibaraki 305-8517 (JP)
(72) Inventor: KAWAGISHI, Makiko, Tsukuba-shi, Ibaraki 305-8634 (JP); SASAKI, Kentaro, Tsukuba-shi, Ibaraki 305-8634 (JP); NUNOME, Tsukasa, Tsukuba-shi, Ibaraki 305-8519 (JP)
(74) Representative: J A Kemp LLP
(86) International application number: PCT/JP2021/012709
(87) International publication number: WO 2021/193865

(57) **Abstract**

The present inventors aimed to identify the gene responsible for the temperature-sensitive male sterility trait in "PL12" and to provide a method for producing conditional male sterile plants targeting the gene, and performed whole genome sequence analysis on "PL12" and the original variety thereof to compare the two. As a result, it was found that there was a deletion of about 150 kb in "PL12" chromosome 7. Then, as for this region, six types of partial deletion lines were produced by genome editing, and the presence or absence of the temperature-sensitive male sterility trait in each line was used as an index to successfully narrow the range, containing the responsible mutation, down to about 10 kb. Furthermore, as a result of creating a line with genome editing-disrupted functions of the two genes located in this region, the gene responsible for the temperature-sensitive male sterility trait was successfully identified. Further, it was confirmed that the functions of the gene could be suppressed to impart the above trait to Arabidopsis thaliana and tomato, as in the case of rice.

## Description

### [Technical Field]

The present invention relates to a method for producing a temperature-sensitive male sterile plant, and the plant obtained thereby. The present invention also relates to a method for producing a hybrid seed using the plant, and the seed obtained thereby.

### [Background Art]

A hybrid provides heterosis in which the growth is vigorous and the yield and quality are high compared to the parent cultivar. Furthermore, hybrid breeding has great advantages, such as the ability to accumulate useful traits based on disease resistance gene clusters and the like. Almost all commonly used vegetables, ornamental plants, corn, and the like are hybrid varieties. Hybrid varieties are also widely used in rice cultivation in China and the United States.

However, since the hybrid variety is a one-generation variety, a large amount of crossing work is required for each seed production, and the labor, time, and cost required for crossing are currently bottlenecks. In general, crossing between crop varieties requires deficient pollens of one variety and attachment of pollens of the other variety. At present, in most cases, either the removal of stamens by hand (emasculation) or the pre-cultivation of special male sterile lines is used to make pollens deficient. For example, since there are no useful male sterile lines in tomatoes, eggplants, and the like, manual crossing with emasculation is performed in hybrid seed production. If male sterile lines are available, reduction of emasculation work can be expected.

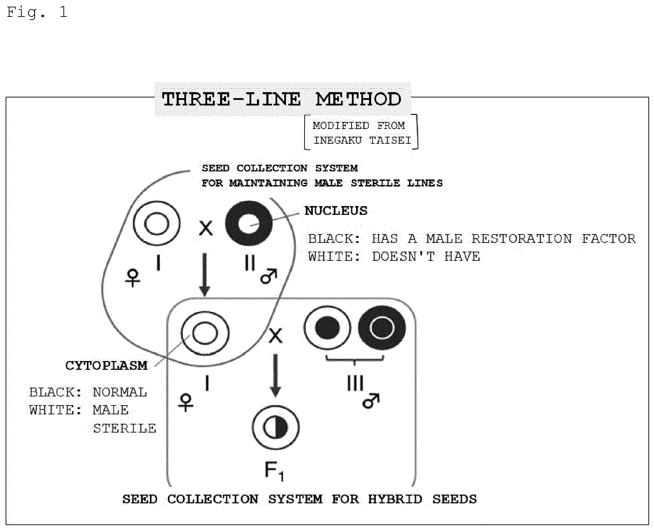

The main method for producing hybrid seeds using male sterile lines is called the three-line method, and requires three lines: a fertility restorer line as a father plant, a male sterile line as a mother plant, and a maintainer line thereof (see Fig. 1). An elite variety to be used requires a special breeding in order to give male sterility and corresponding traits of fertility restoration, which is complicated and takes time. Moreover, there is only a limited number of combinations of available cytoplasmic male sterility factors and their matching fertility restoring factors, and the need to introduce those factors by crossbreeding is laborious and time consuming. This as a result hinders the utilization of a wide range of genetic resources.

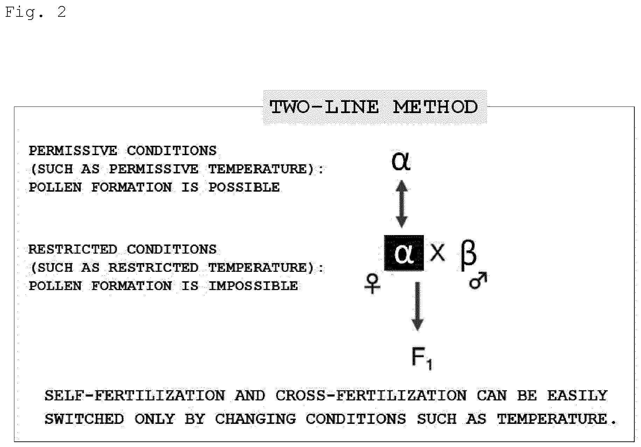

In view of the above, in order to produce hybrid seeds with only two lines (to perform the two-line method), the use of conditional male sterile lines, the use of transgenic lines, and the like have been mainly investigated, as shown in Fig. 2 (PTLs 1 to 3 and NPLs 1 to 3).

For example, an example of male sterility only under certain environmental conditions is temperature-sensitive male sterility. It is possible to control, depending on the temperature conditions, the state in which normal pollen is formed and bears fruit, and the state in which male sterility occurs due to poor pollen formation. For this reason, it is possible to easily switch between self-fertilization and cross-fertilization only by temperature conditions, and there is a great advantage that both the maintenance of the line and the cultivation of the hybrid can be performed in one line. Furthermore, if pollen formation can be controlled with high precision, the possibility of, e.g., crossing using flower-visiting insects such as bees will expand, and improvement in efficiency in seed collection is expected. Therefore, there is a demand for a method for establishing temperature-sensitive male sterile lines in a wide range of crop species and for easy hybrid breeding in a short period of time.

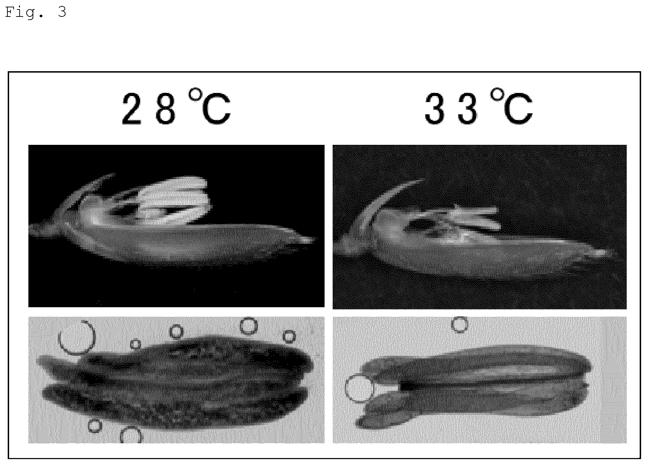

As such a temperature-sensitive male sterile line, in rice, "Rice Norin PL 12" (PL12) whose original variety is "Reimei" shown in Fig. 3 has been reported and used (PTL 1 and NPL 1). However, the gene responsible for the phenotype of this mutant has not yet been identified. Therefore, it has been difficult to produce such temperature-sensitive male sterile lines in varieties other than "Reimei" and in plant species other than rice, and to produce hybrid seeds by the two-line method.

### [Citation List]

### [Patent Literature]

[PTL 1] Japanese Patent Application Publication No. Hei 02-207722
[PTL 2] International Application Japanese-Phase Publication No. 2017-535294
[PTL 3] International Application Japanese-Phase Publication No. 2018-535672

### [Non Patent Literature]

[NPL 1] Yamaguchi et al. 1997, Breeding Science, vol. 47, 371-373
[NPL 2] Chueasiri et al. 2014, PLOS ONE, vol. 9(9), e106386
[NPL 3] Pitnjam et al. 2008, Planta, vol. 228, 813-822

### [Summary of Invention]

### [Technical Problem]

The present invention has been made in view of the above problems, and an object thereof is to identify the gene responsible for the temperature-sensitive male sterility trait in "PL12" and to provide a method for producing conditional male sterile plants targeting the gene.

### [Solution to Problem]

The present inventors first performed fine mapping in order to achieve the above object. However, they could not narrow the range down further from about 1.8 Mb near 54 cM on chromosome 7. Therefore, whole genome sequence analysis was performed on the original variety "Reimei" and "PL12," and the two were compared. As a result, it was found that there was a deletion of about 150 kb in the range of about 1.8 Mb of "PL12."

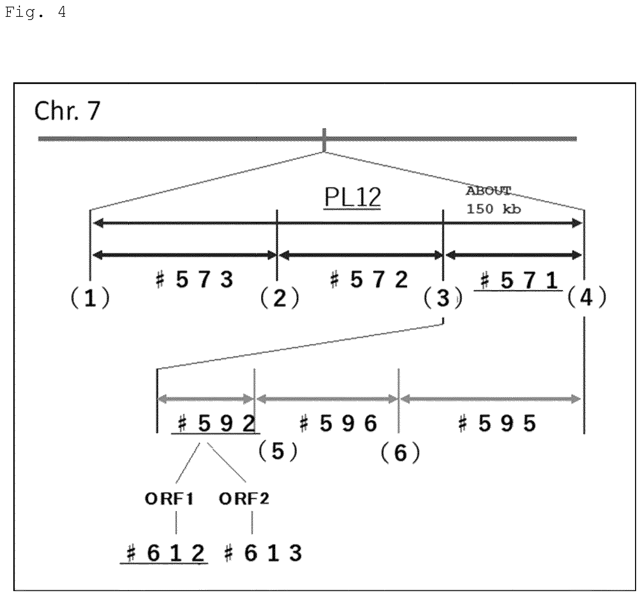

Next, as shown in Fig. 4, the deleted region of about 150 kb was divided into three parts, and genome editing was used to create lines for deleting the respective three regions. As a result, one of these was found to exhibit the same phenotype as "PL12," and a candidate region was narrowed down. Furthermore, in the same manner, the candidate region was divided into three regions, lines for deleting the respective three regions were created, and the genomic region containing the responsible mutation was successfully narrowed down to about 10 kb.

Then, based on the database information, it was predicted that two genes (ORF1 and ORF2 shown in Fig. 4) existed in this 10-kb region, so that genome editing was performed to destroy the function of each gene. As a result, the deletion of ORF1 led to the exhibition of the trait of temperature-sensitive male sterility, and thus it was finally revealed that this gene was responsible for the temperature-sensitive male sterility trait in "PL12" (hereinafter also referred to as "temperature-sensitive male sterility gene").

Furthermore, as a result of suppressing the function of the gene by genome editing, the present inventors found that a similar trait of temperature-sensitive male sterility could be obtained in the dicotyledonous plants Arabidopsis thaliana and tomato, leading to the completion of the present invention. Accordingly, the present invention provides the following.

That is, the present invention relates to a method for producing a temperature-sensitive male sterile plant, and the plant obtained thereby. The present invention also relates to a method for producing a hybrid seed using the plant, and the seed obtained thereby, and more specifically provides the following.
<1> A method for producing a temperature-sensitive male sterile plant, comprising:
   artificially suppressing a function of at least one gene selected from the group consisting of (a) to (d) below
      (a) a gene encoding a protein composed of an amino acid sequence set forth in any one of SEQ ID NOs: 1 to 22,
      (b) a gene encoding a protein composed of an amino acid sequence in which one or more amino acids are substituted, deleted, added, and/or inserted in the amino acid sequence set forth in any one of SEQ ID NOs: 1 to 22,
      (c) a gene encoding an amino acid sequence having 60% or more homology with the amino acid sequence set forth in any one of SEQ ID NOs: 1 to 22, and
      (d) a gene containing a DNA that hybridizes under stringent conditions with a DNA composed of a nucleotide sequence encoding the amino acid sequence set forth in any one of SEQ ID NOs: 1 to 22.
<2> A temperature-sensitive male sterile plant with artificially suppressed functions of at least one gene selected from the group consisting of (a) to (d) below (a) a gene encoding a protein composed of an amino acid sequence set forth in any one of SEQ ID NOs: 1 to 22, (b) a gene encoding a protein composed of an amino acid sequence in which one or more amino acids are substituted, deleted, added, and/or inserted in the amino acid sequence set forth in any one of SEQ ID NOs: 1 to 22,
   (c) a gene encoding an amino acid sequence having 60% or more homology with the amino acid sequence set forth in any one of SEQ ID NOs: 1 to 22, and
   (d) a gene containing a DNA that hybridizes under stringent conditions with a DNA composed of a nucleotide sequence encoding the amino acid sequence set forth in any one of SEQ ID NOs: 1 to 22.
<3> A method for producing hybrid seeds comprising:
   cross-pollinating the temperature-sensitive male sterile plant according to <2>, cultivated under a restricted temperature and made male-sterile, with a given plant; and
   collecting seeds from the temperature-sensitive male sterile plant.
<4> A hybrid seed comprising:
   the temperature-sensitive male sterile plant according to <2>, cultivated under a restricted temperature and made male-sterile, as a mother plant; and
   a given plant as a father plant.

### [Advantageous Effects of Invention]

The present invention makes it possible to produce temperature-sensitive sterile plants. In particular, temperature-sensitive sterile plants can be produced without requiring special breeding other than suppressing the function of the temperature-sensitive male sterility gene of the present invention.

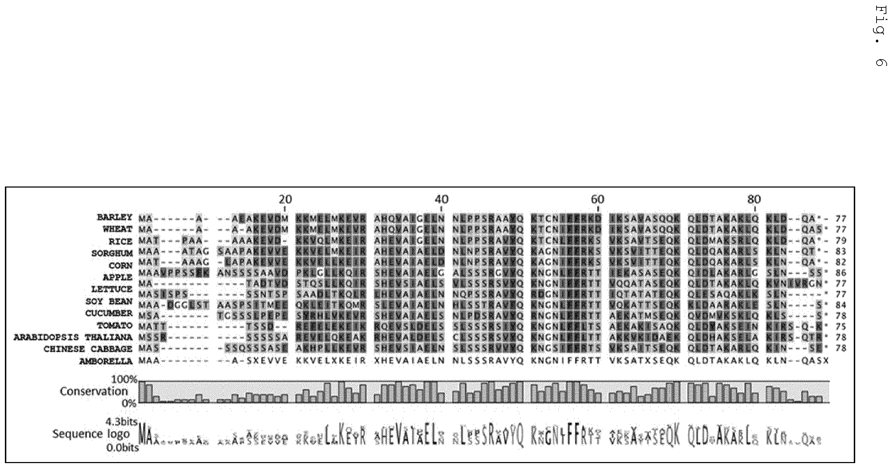

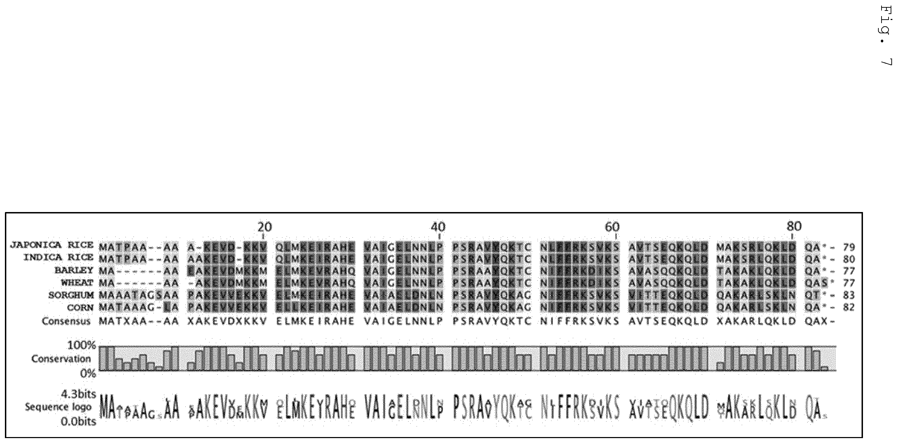

Moreover, as shown in Figs. 6 and 7, the amino acid sequences of the proteins encoded by the temperature-sensitive male sterility gene of the present invention are highly conserved. This gene also exists in Amborellales, which is the most primitive angiosperm, and thus is understood as a gene that exists at least in common among angiosperms. Therefore, it is considered that this gene can be used in all crops in principle. As described above, in the method of the present invention, since the lines to be used are not limited, it is possible to produce male sterile lines by suppressing the function of this gene in myriad varieties and lines of myriad plant species.

Then, by cultivating the obtained temperature-sensitive male sterile plant as a mother plant together with another line as a father plant under a restricted temperature, it is possible to easily obtain hybrid seeds in which these two lines are crossed.

### [Brief Description of Drawings]

[Fig. 1] Fig. 1 is a diagram showing an outline of the three-line method used as a method for producing hybrid seeds.
[Fig. 2] Fig. 2 is a diagram showing an outline of the two-line method used as a method for producing hybrid seeds. "α" indicates a line that becomes male sterile depending on environmental conditions such as temperature, and "β" indicates a given variety or line.
[Fig. 3] Fig. 3 is a photograph showing the phenotype of "PL12". It shows an image of the flower (upper) at the flowering stage and the staining image of the anthers (lower) at the permissive temperature (28°C) and the restrictive temperature (33°C).
[Fig. 4] Fig. 4 is a schematic diagram showing the process of identifying the responsible factor of "PL12". First, guide RNAs were designed at sites (1) to (6). The deletion region of the genome of the temperature-sensitive male sterile line "PL12" was used as a start to create a line with a partial deletion by a method of deleting between two double-strand breaks with CRISPR/Cas9. Candidate regions with the same phenotype as that of "PL12" were sequentially narrowed down. It was found that line #571 lacking between (3) and (4) had the trait of temperature-sensitive male sterility, so that its interior was subdivided and analyzed, narrowing the candidate down in between (3) to (5). There were two genes in this region, ORF1 and ORF2, and a mutation was introduced to delete each ORF. Note that in the figure, the underscored line names are lines having the trait of temperature-sensitive male sterility.

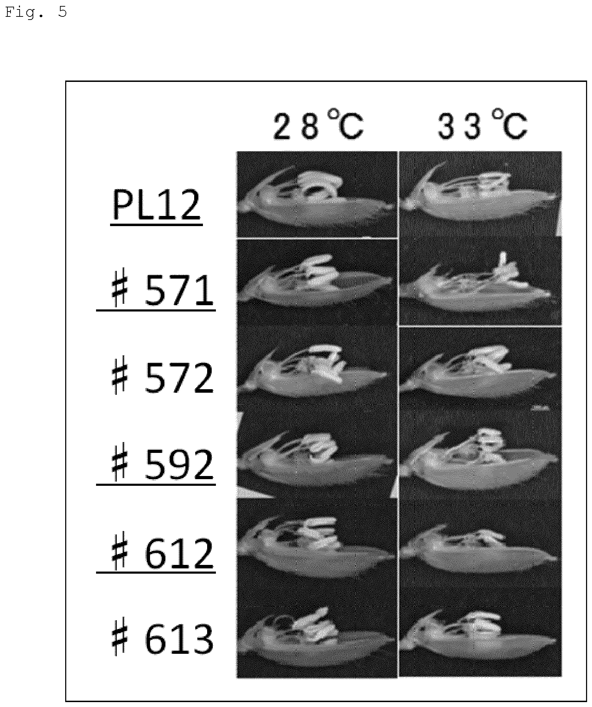

[Fig. 5] Fig. 5 is a photograph showing an image of the flower (representative examples) at the flowering stage at the permissive temperature (28°C) and the restrictive temperature (33°C) of each line with a partial deletion shown in Fig. 4. In the figure, the underscored lines #571, #592, and #612 exhibited the trait of temperature-sensitive male sterility.
[Fig. 6] Fig. 6 shows an alignment comparing the amino acid sequences encoded by the temperature-sensitive male sterility gene of the present invention among plant species. Based on the information in the higher plant database, ORFs were predicted and translated into amino acid sequences for comparison. The QIAGEN CLC software was used to perform sequence analysis.
[Fig. 7] Fig. 7 shows an alignment comparing the amino acid sequences encoded by the temperature-sensitive male sterility gene of the present invention among rice crops. Based on the information in the rice crop database, ORFs were predicted and translated into amino acid sequences for comparison. The QIAGEN CLC software was used to perform sequence analysis.

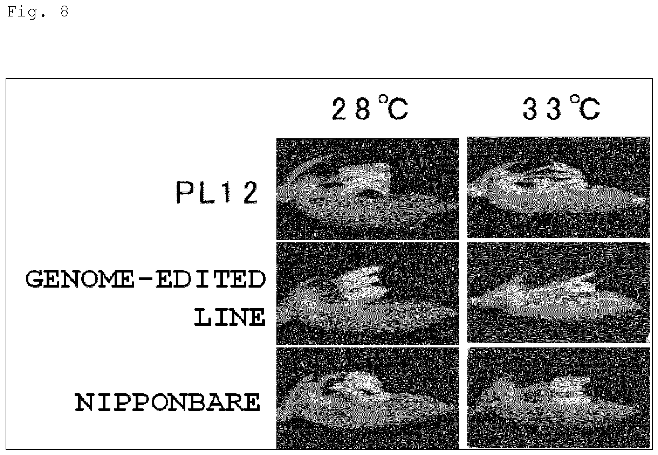

[Fig. 8] Fig. 8 is a photograph showing phenotypes (representative examples) of a genome-edited line and the like of rice. "PL12" normally forms pollen at 28°C and bears yellow and full anthers, but at 33°C does not develop pollen and bears anthers with abnormal morphology and color. The standard variety " Nipponbare " forms pollen normally regardless of the temperature. A line lacking the gene in " Nipponbare " by genome editing exhibits the phenotype of temperature-sensitive male sterility as in "PL12."

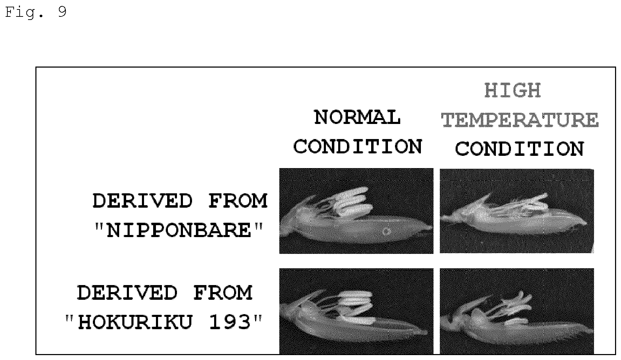

[Fig. 9] Fig. 9 is a photograph showing the appearance (representative examples) of a genome-edited line and the like of rice. A line of rice variety "Nipponbare" whose gene function had been disrupted by genome editing was cultivated at 28°C (left) and 33°C (right). At 28°C, the fruit is normally borne and the ears are drooping, but at 33°C, no fruit is borne and the ears are standing.

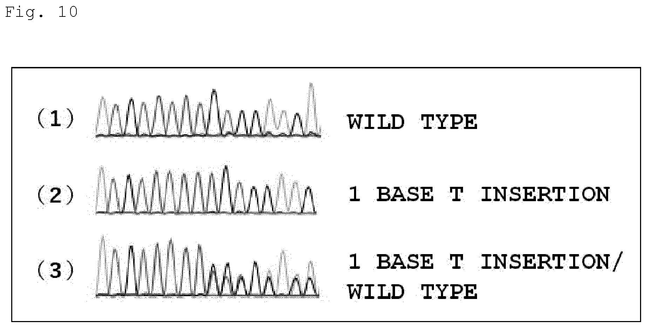

[Fig. 10] Fig. 10 is a photograph showing the appearance (representative examples) of a genome-edited line and the like of "Nipponbare" and "Hokuriku 193." As in the case of the genome-edited line derived from "Nipponbare," the genome-edited line derived from "Hokuriku 193" also forms pollen and develops anthers under normal conditions, but becomes male sterile under high temperature conditions due to deficiency in pollen formation.

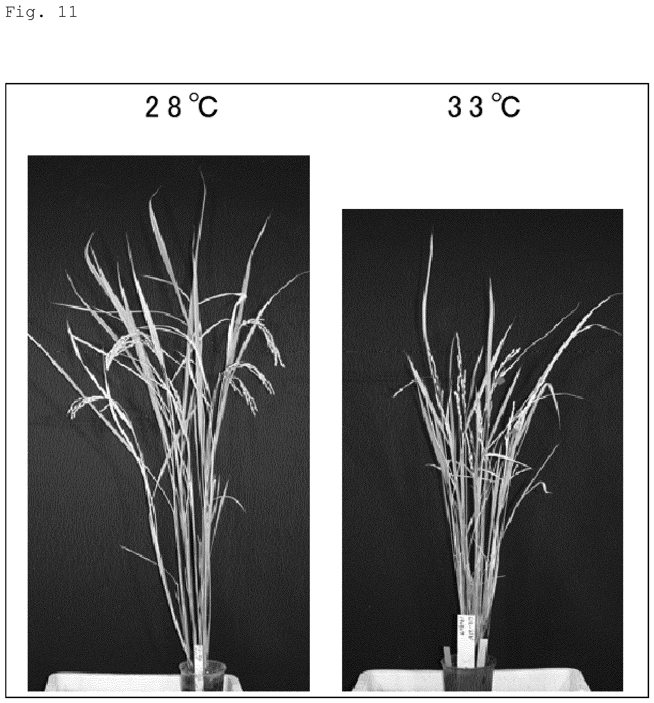

[Fig. 11] Fig. 11 shows the results of purifying genomic DNA from each of (1) "Oonari," (2) the genome-edited line derived from "Hokuriku 193," and (3) the individuals obtained by applying the pollen of "Oonari" to the genome-edited line derived from "Hokuriku 193" cultivated at high temperature, and analyzing the nucleotide sequence of s mutation site of the temperature-sensitive male sterility gene of the present invention. As shown below, (3) is a heterozygote of the genomic sequences of (1) and (2), indicating that a hybrid was obtained by crossing (1) and (2). (1) ACGCTCTTGCGGAAGA (SEQ ID NO: 34) wild type, (2) ACGCTCTTTGCGGAAGA (SEQ ID NO: 35) 1 base T insertion, and (3) ACGCTCTT (T/G) (G/C) (C/G) G (G/A) A (A/G) (G/A) (SEQ ID NO: 36) 1 base T insertion/wild type.

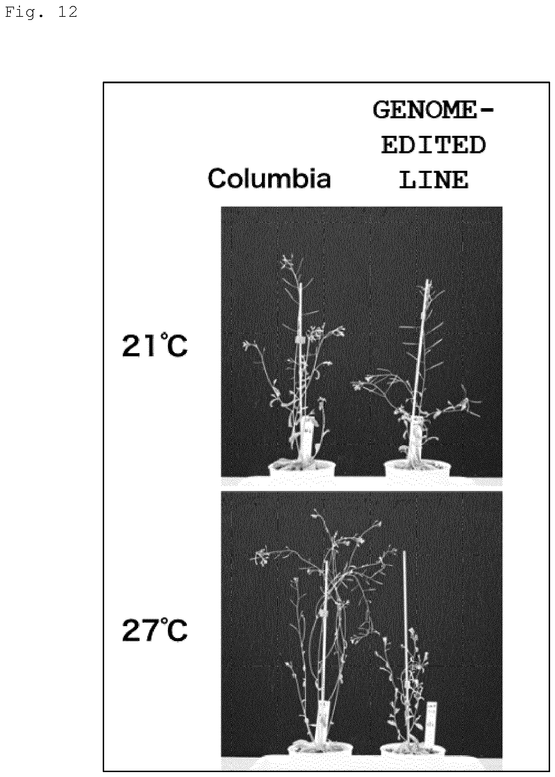

[Fig. 12] Fig. 12 is a photograph showing the appearance (representative examples) of a genome-edited line of Arabidopsis thaliana. The lines of Arabidopsis thaliana whose gene function had been disrupted by genome editing were cultivated at 21°C (upper) and 27°C (lower). At 21°C, the fruit is normally borne and the pods are the same in length as the wild type, but at 27°C, no fruit is borne and the pods do not elongate.

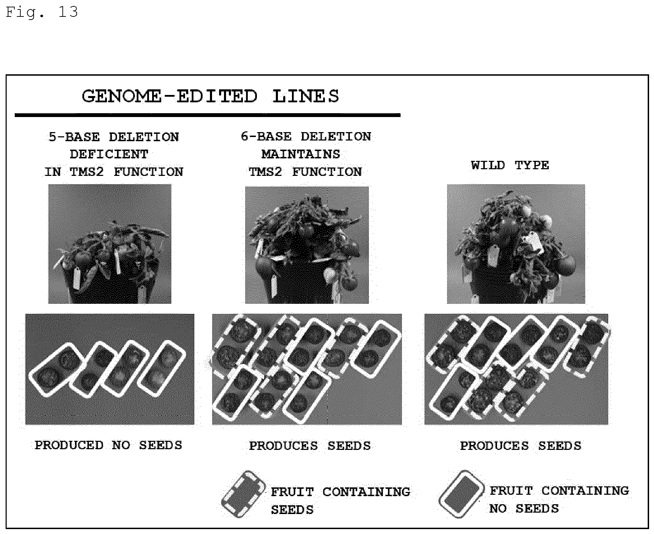

[Fig. 13] Fig. 13 is a photograph showing the results of cultivating genome-edited tomato lines together with a wild type under high-temperature conditions (30°C during the day/28°C at night) and determining whether seeds were formed in the resulting fruits. In the figure, the upper part shows the individual plants bearing fruits, and the lower part shows photographs of the fruits split in half. Seeded fruits are shown with dotted lines and seedless fruits with solid lines. In the figure, "TMS2" means the temperature-sensitive male sterility gene of the present invention (see SEQ ID NOs: 43 and 44) . In the line with a 6-base deletion mutation, despite the deletion of two amino acids at the relevant sites (see SEQ ID NOs: 45 and 46), it is considered that a functional protein is still formed, and there is no difference in seed formation from the wild type. In contrast, it was shown that the line with a 5-base deletion mutation (see SEQ ID NOs: 47 and 48) presumed to impair protein function due to frameshifting could not produce seeds under high-temperature conditions.

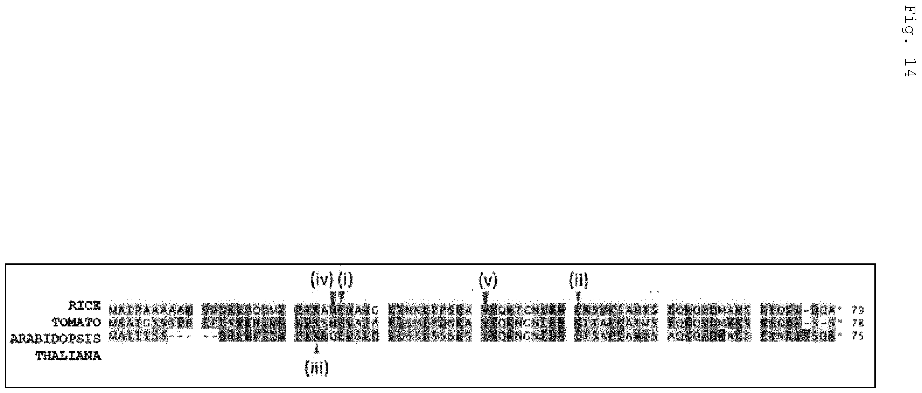

[Fig. 14] Fig. 14 is a schematic diagram showing amino acid sequences encoded by the temperature-sensitive male sterility genes derived from rice, tomato, and Arabidopsis thaliana, and mutation sites in genome-edited individuals prepared in Examples described later. In the figure, (i) and (ii) indicate the mutation introduction sites in rice (positions where double-strand breaks were introduced with CRISPR/Cas9), (iii) indicates the mutation introduction site in Arabidopsis thaliana, and (iv) and (v) indicate the mutation introduction sites in tomato.

### [Description of Embodiments]

### (Method for Producing Temperature-Sensitive Male Sterile Plants)

As shown in Examples below, the present inventors have elucidated a gene responsible for the phenotype of the temperature-sensitive male sterile line, "Rice Norin PL 12" (PL12). Furthermore, in wild-type rice, Arabidopsis thaliana, and tomato, the present inventors have also succeeded in conferring these plants with temperature-sensitive male sterility by suppressing the function of the gene by genome editing. Therefore, the method for producing a temperature-sensitive male sterile plant of the present invention is characterized by including artificially suppressing the function of the gene (temperature-sensitive male sterile gene), and more specifically provides the following.

A method for producing a temperature-sensitive male sterile plant, comprising:
artificially suppressing a function of at least one gene selected from the group consisting of (a) to (d) below
(a) a gene encoding a protein composed of an amino acid sequence set forth in any one of SEQ ID NOs: 1 to 22,
(b) a gene encoding a protein composed of an amino acid sequence in which one or more amino acids are substituted, deleted, added, and/or inserted in the amino acid sequence set forth in any one of SEQ ID NOs: 1 to 22,
(c) a gene encoding an amino acid sequence having 60% or more homology with the amino acid sequence set forth in any one of SEQ ID NOs: 1 to 22, and
(d) a gene containing a DNA that hybridizes under stringent conditions with a DNA composed of a nucleotide sequence encoding the amino acid sequence set forth in any one of SEQ ID NOs: 1 to 22.

In the present invention, "temperature-sensitive male sterile" means a trait that normally bears fruit when cultivated under permissive temperature conditions described later, but becomes male sterile when cultivated under restricted temperature conditions described later.

In the present invention, the "plant" to be imparted with the temperature-sensitive male sterility trait is not particularly limited as long as it has the temperature-sensitive male sterility gene described later, and examples thereof include angiosperms, including monocotyledons (for example, Gramineae plants such as rice, barley, wheat, sorghum, and corn, and Amaryllidaceae plants such as onions and Welsh onions) and dicotyledons (for example, cruciferous plants such as Arabidopsis thaliana, Chinese cabbage, Brassica napus (rapeseed), and Brassica oleracea L. (progenitors of cabbage, cauliflower, broccoli, and the like), solanaceous plants such as tomatoes and potatoes, Asteraceae plants such as lettuce and sunflowers, leguminous plants such as soybeans, cucurbitaceous plants such as cucumbers, apiaceous plants such as carrots, rosaceous plants such as apples, Musaceae plants such as bananas, Amborellaceae plants (Amborella trichopoda)), gymnosperms, bryophytes, and ferns. Furthermore, genetically-modified and genome-edited versions of these plants (such as herbicide-tolerant crops, pest-tolerant crops, disease-resistant crops, taste-improved crops, storage stability-improved crops, and yield-improved crops) may also be used.

Examples of genes encoding typical amino acid sequences derived from various plant species, as the "temperature-sensitive male sterility gene" whose function is to be suppressed in the present invention, are as shown in Table 1 below.

**[Table 1]**

| Species Name | Amino Acid Sequence (SEQ ID NO:) |
|---|---|
| Rice (Japonica Type) | 1 |
| Rice (Indica Type) | 2 |
| Barley | 3 |
| Wheat | 4 |
| Sorghum. | 5 |
| Corn | 6 |
| Soy Bean | 7 |
| Potato | 8 |
| Tomato | 9 |
| Cucumber | 10 |
| Banana | 11 |
| Sunflower | 12 |
| Carrot | 13 |
| Arabidopsis Thaliana | 14 |
| Lettuce | 15 |
| Apple | 16 |
| Chinese Cabbage | 17 |
| Onion | 18 |
| Welsh Onion | 19 |
| Brassica Napus | 20 |
| Brassica Oleracea L. | 21 |
| Amborella trichopoda | 22 |

Note that nucleotide sequences could also mutate in the natural world. The encoded amino acid could change accordingly. Therefore, the temperature-sensitive male sterility gene of the present invention, as long as it can confer the trait of temperature-sensitive male sterility by the suppression of its function, also includes a gene encoding a protein composed of an amino acid sequence in which one or more amino acids are substituted, deleted, added, and/or inserted in the amino acid sequence set forth in any one of SEQ ID NOs: 1 to 22.

Here, "more" means usually 50 amino acids or less, preferably 45 amino acids or less, more preferably 40 amino acids or less, further preferably 35 amino acids or less, more preferably 30 amino acids or less, further preferably 25 amino acids or less, more preferably 20 amino acids or less, further preferably 15 amino acids or less, more preferably 10 amino acids or less (for example, 9 amino acids or less, 8 amino acids or less, 7 amino acids or less, and 6 amino acids or less), and particularly preferably a few amino acids or less (for example, 5 amino acids or less, 4 amino acids or less, 3 amino acids or less, and 2 amino acids or less).

Furthermore, in the current state of the art, when a particular gene is obtained, those skilled in the art can use the nucleotide sequence information of that gene to identify its homologous genes from the same species or other plants. Examples of the method for identifying homologous genes include a hybridization technique (Southern, E. M., J. Mol. Biol., 98: 503, 1975) and polymerase chain reaction (PCR) techniques (Saiki, R. K., et al. Science, 230: 1350-1354, 1985, Saiki, R. K. et al. Science, 239: 487-491, 1988). To identify homologous genes, hybridization reactions are typically performed under stringent conditions. Examples of stringent hybridization conditions include 6 M urea, 0.4% SDS, and 0.5 x SSC conditions, or equivalent-stringency hybridization conditions. Higher-stringency conditions, for example, 6 M urea, 0.4% SDS, and 0.1 x SSC, are expected to isolate genes with higher homology. The temperature-sensitive male sterility gene of the present invention, as long as it can confer the trait of temperature-sensitive male sterility by the suppression of its function, includes a gene containing a DNA that hybridizes under stringent conditions with a DNA composed of a nucleotide sequence encoding the amino acid sequence set forth in any one of SEQ ID NOs: 1 to 22.

The protein encoded by the identified homologous gene usually has high homology (high similarity), preferably high identity, with that encoded by the above particular gene. Here, "high" means at least 40% or more, more preferably 50% or more, further preferably 60% or more, more preferably 70% or more, further preferably 80% or more, and more preferably 85% or more (for example, 90% or more, 95% or more, 96% or more, 97% or more, 98% or more, and 99% or more). The temperature-sensitive male sterility gene of the present invention, as long as it can confer the trait of temperature-sensitive male sterility by the suppression of its function, includes a gene encoding an amino acid sequence having 60% or more homology (similarity) or 40% or more identity with the amino acid sequence set forth in any one of SEQ ID NOs: 1 to 22. Note that the homology of the amino acid sequence set forth in SEQ ID NO: 14 with that set forth in SEQ ID NO: 1 is 65% (45% identity), and the homology of the amino acid sequence set forth in SEQ ID NO: 14 with that set forth in SEQ ID NO: 9 or 44 is 73% (55% identity).

Sequence homology can be determined using the BLAST program (Altschul et al. J. Mol. Biol., 215: 403-410, 1990). The program is based on the algorithm BLAST by Karlin and Altschul (Proc. Natl. Acad. Sci. USA, 87: 2264-2268, 1990, Proc. Natl. Acad. Sci. USA, 90: 5873-5877, 1993). For instance, in the analysis of an amino acid sequence by BLAST, the parameters are score = 50 and wordlength = 3, for example. In addition, in the analysis of an amino acid sequence using the Gapped BLAST program, it can be performed as described in Altschul et al. (Nucleic Acids Res. 25: 3389-3402, 1997). When using BLAST and Gapped BLAST programs, the default parameters of each program are used. Specific methods of these analysis methods are known.

The "artificial suppression of the function of the temperature-sensitive male sterility gene" of the present invention includes both complete suppression (inhibition) and partial suppression of the function. In addition to the artificial suppression of the expression of the temperature-sensitive male sterility gene, the artificial suppression of the activity of the protein encoded by the temperature-sensitive male sterility gene is also included. Such artificial suppression can be performed, for example, by introducing mutations into the coding region, noncoding region, transcription control region (promoter region), and the like of the temperature-sensitive male sterility gene.

In the present invention, the mutation introduced into the temperature-sensitive male sterility gene is not particularly limited as long as it suppresses the function of the gene, and examples thereof include nucleotide substitutions, deletions, additions, and/or insertions, preferably nonsense mutations, frameshift mutations, and null mutations. Also, the number of mutations introduced into the temperature-sensitive male sterility gene is not particularly limited either as long as it suppresses the function of the gene, and may be one or more (for example, 2, 3 or less, 5 or less, 10 or less, 20 or less, 30 or less, 40 or less, or 50 or less).

Examples of such mutations include nucleotide mutations with alterations or deletions of amino acids after position 53 (about 33% of the total) of the amino acid sequence set forth in SEQ ID NO: 1, as shown in Table 2 below. Further, as shown in Table 3 below, examples also include nucleotide mutations with alterations or deletions of amino acids after position 18 or 19 (about 76% of the total) set forth in SEQ ID NO: 14. Further examples include nucleotide mutations with alterations or deletions of amino acids after position 24 (about 70% of the total) set forth in SEQ ID NO: 9 or 44. Such deletion suppresses the function of the temperature-sensitive male-sterile gene of the present invention, making it possible to obtain a temperature-sensitive male sterile plant.

Therefore, the mutation introduced into the temperature-sensitive male sterility gene does not have to delete the entire amino acid sequence of the protein encoded by the gene, and may be introduced into the gene so as to delete or alter a portion thereof.

Note that when a portion of the expressed protein is deleted due to genetic mutation, it usually suffices that 10% or more of the total amino acids may be altered or deleted, preferably 20% or more, preferably 20% or more, further preferably 25% or more, more preferably 30% or more, further preferably 35% or more, more preferably 40% or more, further preferably 45% or more, more preferably 50% or more, further preferably 55% or more, more preferably 60% or more, further preferably 65% or more, more preferably 70% or more, further preferably 75% or more, more preferably 80% or more, further preferably 85% or more, more preferably 90% or more, and further preferably 95% or more (for example, 96% or more, 97% or more, 98% or more, 99% or more) may be altered or deleted.

The region whose amino acid sequence is to be altered or deleted in this manner is preferably the C-terminal region, as shown in Examples below. Further, Figs. 6 and 7 also suggest a highly conserved region in the amino acid sequence encoded by the temperature-sensitive male sterility gene of the present invention, that is, a region important for its function. Therefore, such conserved regions are also suitable as regions whose amino acid sequences are to be altered or deleted. Examples of conserved regions include the region composed of positions 10 to 75 in the amino acid sequence set forth in SEQ ID NO: 1, or a region corresponding thereto. Note that the corresponding region is a region that is aligned in the same row with the aforementioned region in the amino acid sequence set forth in SEQ ID NO: 1 when nucleotide and amino acid sequence analysis software (such as GENETYX-MAC or Sequencher) or BLAST (http://blast.ncbi.nlm.nih.gov/Blast.cgi) is used to align the amino acid sequence set forth in SEQ ID NO: 1 with amino acid sequences derived from other plants (for example, SEQ ID NOs: 2 to 21) as shown in Figs. 6, 7, and 14.

Introduction of mutations into the temperature-sensitive male sterility gene can be achieved by a method known to those skilled in the art. Such known methods include, but are not limited to, genome editing, physical mutagenesis, methods using chemical mutagens, a method of introducing a transposon or the like into genomic DNA, and methods that target transcription products using siRNA, antisense RNA, RNA with ribozyme activity, and the like.

Among these, genome editing, the methods that target transcription products, and the TILLING method described later are preferable from the viewpoint that mutation can be artificially introduced by targeting the temperature-sensitive male sterility gene.

Genome editing is a method of modifying a target gene using a site-specific nuclease (such as zinc finger nuclease (ZFN), transcription activator-like effector nuclease (TALEN), or DNA double-strand cleavage enzyme such as CRISPR-Cas9). Examples thereof include methods that utilize fusion proteins such as ZFNs (US Patent Nos. 6265196, 8524500, 7888121, and European Patent No. 1720995), TALENs (US Patent No. 8470973 and US Patent No. 8586363), PPRs with fused nuclease domains (pentatricopeptide repeat) (Nakamura et al., Plant Cell Physiol 53: 1171-1179 (2012)) and complexes of guide RNA and protein such as CRISPR-Cas9 (US Patent No. 8697359 and International Publication No. WO2013/176772), CRISPR-Cpf1 (Zetsche B. et al., Cell, 163 (3): 759-71, (2015)), and Target-AID (K. Nishida et al., Targeted nucleotide editing using hybrid prokaryotic and vertebrate adaptive immune systems, Science, DOI:10.1126/science.aaf8729, (2016)).

Examples of physical mutagenesis include heavy ion beam (HIB) irradiation, fast neutron beam irradiation, gamma ray irradiation, and ultraviolet irradiation (see Hayashi et al., Cyclotrons and Their Applications, 2007, 18th Int. Conf., pp. 237 to 239, and Kazama et al., Plant Biotechnology, 2008, vol. 25, pp. 113 to 117). Examples of the methods using chemical mutagens include a method of treating seeds and the like with a chemical mutagen (see Zwar and Chandler, Planta, 1995, vol. 197, pp. 39 to 48, etc.). The chemical mutagens are not particularly limited, but examples thereof ethyl methanesulfonate (EMS), N-ethyl-N-nitrosourea (ENU), N-methyl-N-nitrosourea (MNU), sodium azide, sodium bisulfite, hydroxylamine, N-methyl-N'-nitro-N-nitroguanidine (MNNG), N-methyl-N'-nitrosoguanidine (NTG), O-methylhydroxylamine, nitrous acid, formic acid, and nucleotide analogues.

Examples of the methods of introducing transposons and the like into genomic DNA include methods of inserting transposons such as TOS17, T-DNA, and the like into genomic DNA of plants (see Kumar et al., Trends Plant Sci., 2001, vol. 6, issue 3, pp. 127 to 134, and Tamara et al., Trends in Plant Science, 1999, vol. 4, issue 3. pp. 90 to 96).

For plants introduced with mutations by the above methods, it is possible to confirm that mutations have been introduced into the temperature-sensitive male sterility gene by known methods. Examples of such known methods include DNA sequencing (such as next-generation sequencing), PCR, analysis using microarrays, Southern blotting, and Northern blotting. According to these methods, whether or not a mutation has been introduced into the temperature-sensitive male sterility gene can be determined by comparing the sequences or lengths of the gene before and after the mutation introduction. In addition, in plants with mutations introduced into the transcriptional regulatory region or the like by using Northern blotting, RT-PCR, Western blotting, ELISA, analysis using microarrays, or the like, if a decrease in the expression levels of the transcription products or translation products of the temperature-sensitive male sterility gene is observed, one can confirm that the plant has a mutation introduced into the temperature-sensitive male sterility gene.

Further, another method for confirming the introduction of mutations into temperature-sensitive male sterility genes is TILLING (Targeting Induced Local Lesions in Genomes) (see Slade et al., Transgenic Res., 2005, vol. 14, pp. 109 to 115, and Comai et al., Plant J., 2004, vol. 37, pp. 778 to 786). In particular, when random mutations have been introduced into a plant genome using the aforementioned heavy ion beam irradiation, chemical mutagen, or the like, a temperature-sensitive male sterility gene or a portion thereof can be amplified by PCR, and then individuals having mutations in the amplified products can be selected by the aforementioned TILLING or the like.

In addition, mutations introduced into genes other than the target gene can also be removed by crossing a plant introduced with a mutation by the above-described method and a wild-type plant, followed by backcrossing.

A plant that has a mutation introduced into the temperature-sensitive male sterility gene and thus has suppressed functions of the gene may be a heterozygote of the temperature-sensitive male sterility gene. In that case, for example, such heterozygotes are crossed to obtain F1 plants, and from these F1 plants, homozygotes having the temperature-sensitive male sterility gene introduced with that mutation are selected. In this case, the "plant that is a homozygote having the temperature-sensitive male sterility gene introduced with that mutation" includes not only a plant that has two alleles of a temperature-sensitive male sterility gene having mutations identical to each other, but also a plant that has a first temperature-sensitive male sterility gene encoding a protein having a first mutation and having suppressed activity and a second temperature-sensitive male sterility gene encoding a protein having a second mutation and having suppressed activity.

In the present invention, Examples of the method for artificially suppressing the function of the temperature-sensitive male sterility gene also include the above-described mutation introductions as well as methods that target transcription products of temperature-sensitive male sterility genes, such as a method that uses DNA encoding a dsRNA (double-stranded RNA, such as siRNA) complementary to the transcription products of a temperature-sensitive male sterility gene, a method that uses DNA encoding an antisense RNA complementary to the transcription products of a temperature-sensitive male sterility gene (antisense DNA), and a method that uses DNA encoding an RNA having ribozyme activity that specifically cleaves the transcription products of a temperature-sensitive male sterility gene (ribozyme method).

In the present invention, the artificial suppression of temperature-sensitive male sterility gene functions can be performed on various plants, seeds, or plant cells according to the methods described above. Plant cells include plant-derived cultured cells as well as cells in plants. Further, various forms of plant-derived cells, such as suspension cultured cells, protoplasts, leaf explants, callus, immature embryos, and pollen are included.

In addition, in the present invention, a DNA encoding a site-specific nuclease, a fusion protein, or a guide RNA-protein complex, a DNA encoding a transposon, a DNA encoding a double-stranded RNA, a DNA encoding an antisense RNA, a DNA encoding an RNA having ribozyme activity, or the like, as described above, may be introduced into a plant cell in the form of a vector insert.

The vector to be inserted with the DNA for artificially suppressing the function of the temperature-sensitive male sterility gene is not particularly limited as long as the inserted gene can be expressed in plant cells, and may contain a promoter for constitutively or inducibly expressing the DNA. Examples of promoters for constitutive expression include rice ubiquitin promoter, cauliflower mosaic virus 35S promoter, rice actin promoter, corn ubiquitin promoter, and the like. In addition, examples of promoters for inducible expression include promoters known to be expressed by exogenous factors such as infection or invasion of filamentous fungi, bacteria, and viruses, low temperature, high temperature, dryness, irradiation of ultraviolet rays, and spraying of specific compounds. Furthermore, U6 promoters and pol III promoters are preferably used as promoters for expressing DNAs encoding short RNAs such as guide RNAs and siRNAs as the DNAs of the present invention.

Various methods known to those skilled in the art can be used to introduce the DNA or a vector inserted with the DNA into a plant cell, such as the particle gun method, the particle bombardment method, the method via Agrobacterium (Agrobacterium method), the polyethylene glycol method, and the electroporation method.

In addition, even in the form of non-DNA, proteins such as site-specific nucleases, fusion proteins, and transposons described above or RNAs such as guide RNAs, double-stranded RNAs, antisense RNAs, and RNAs having ribozyme activity described above can be introduced into plant cells to introduce mutations.

Also, temperature-sensitive male sterile plants can be obtained by regenerating plants from plant cells whose gene functions have been artificially suppressed by the above-described methods or the like.

For example, for rice, several techniques have already been established for producing transformed plants and are widely used in the technical field of the present invention, such as a method of introducing genes into a protoplast with polyethylene glycol and regenerating the plant (Datta, S. K. In Gene Transfer To Plants (Potrykus I and Spangenberg Eds.) pp 66-74, 1995), a method of introducing genes into a protoplast by electric pulse and regenerating the plant (Toki et al. Plant Physiol. 100, 1503-1507, 1992), a method of directly introducing genes into cells by the particle gun method and regenerating the plant (Christou et al. Bio/technology, 9: 957-962, 1991), a method of introducing genes via Agrobacterium and regenerating the plant (Hiei et al. Plant J. 6: 271-282, 1994), and the like.

For Arabidopsis thaliana, the floral dip method (Clough SJ & Bent AF, Plant J 16: 735-743, 1998) and method of Akama et al.(Akama et al. Plant Cell Reports 12: 7-11, 1992) can be mentioned, and these methods can be preferably used in the present invention.

In addition, as the method for producing transformed plants of wheat, the methods described by Tingay et al. (Tingay S.et al. Plant J. 11: 1369-1376, 1997), Murray et al. (Murray F et al. Plant Cell Report 22: 397-402, 2004), and Travalla et al. (Travalla S et al. Plant Cell Report 23: 780-789, 2005) can be mentioned.

Preferably used examples of the method of regenerating sorghum plants include a method of regenerating a plant by introducing genes into immature embryos or callus by the Agrobacterium method or the particle gun method, and a method of pollination using genetically introduced pollen by ultrasonic (J. A. Able et al., In Vitro Cell. Dev. Biol. 37: 341-348, 2001, A. M. Casas et al., Proc. Natl. Acad. Sci. USA 90: 11212-11216, 1993, V. Girijashankar et al., Plant Cell Rep 24: 513-522, 2005, J. M. JEOUNG et al., Hereditas 137: 20-28, 2002, V Girijashankar et al., Plant Cell Rep 24 (9): 513-522, 2005, Zuo-yu Zhao et al., Plant Molecular Biology 44: 789-798, 2000, S. Gurel et al., Plant Cell Rep 28 (3): 429-444, 2009, ZY Zhao, Methods Mol Biol, 343: 233-244, 2006, AK Shrawat and H Lorz, Plant Biotechnol J, 4 (6): 575-603, 2006, D Syamala and P Devi Indian J Exp Biol, 41 (12): 1482-1486, 2003, Z Gao et al., Plant Biotechnol J, 3 (6): 591-599, 2005).

For corn, the method described in Shillito et al. (Bio/Technology, 7: 581, 1989) and the method described in Gorden-Kamm et al. (Plant Cell 2: 603, 1990) can be mentioned.

For tomatoes, the method described in Matsukura et al. (J. Exp. Bot., 44: 1837-1845, 1993) can be mentioned.

For soybeans, the method described in a patent publication (US Patent No. 5,416,011) can be mentioned.

For potatoes, the method described in Visser et al. (Theor. Appl. Genet, 78: 594, 1989) can be used.

Other plants can also be transformed and regenerated into plants using the method described in Tabei et al. (edited by Yutaka Tabei, "Protocols for Plant Transformation," Kagaku-Dojin Publishing, published on September 20, 2012).

### (Temperature-Sensitive Male Sterile Plant and Use Thereof)

The methods described above and the like make it possible to obtain a temperature-sensitive male sterile plant with artificially suppressed functions of the temperature-sensitive male sterility gene of the present invention. Accordingly, the present invention provides a temperature-sensitive male sterile plant with artificially suppressed functions of at least one gene selected from the group consisting of (a) to (d) below
(a) a gene encoding a protein composed of an amino acid sequence set forth in any one of SEQ ID NOs: 1 to 22,
(b) a gene encoding a protein composed of an amino acid sequence in which one or more amino acids are substituted, deleted, added, and/or inserted in the amino acid sequence set forth in any one of SEQ ID NOs: 1 to 22,
(c) a gene encoding an amino acid sequence having 60% or more homology with the amino acid sequence set forth in any one of SEQ ID NOs: 1 to 22, and
(d) a gene containing a DNA that hybridizes under stringent conditions with a DNA composed of a nucleotide sequence encoding the amino acid sequence set forth in any one of SEQ ID NOs: 1 to 22.

Note that the temperature-sensitive male sterility gene, artificial suppression of the function thereof, the plant to be conferred with temperature-sensitive male sterility by the suppression, and the like are as described above, and the temperature-sensitive male sterile plant of the present invention is preferably a plant other than "PL12" (Rice Norin PL 12), and more preferably a plant other than "Reimei" with artificially suppressed functions of the temperature-sensitive male sterility gene of the present invention.

Moreover, once a plant with artificially suppressed functions of the temperature-sensitive male sterility gene is obtained, offspring can be obtained from the plant by sexual or asexual reproduction. Furthermore, it is also possible to obtain propagation materials (such as seeds, cuttings, stumps, callus, and protoplasts) from the plant, progeny, or clones thereof, and mass-produce the plant based on these materials. Thus, the present invention includes progeny and clones of temperature-sensitive male sterile plants and propagation materials thereof. Examples of propagation materials include seeds, strains, callus, and protoplasts.

Also, the temperature-sensitive male sterile plant of the present invention can be used in a method for producing hybrid seeds by the two-line method as shown in Fig. 2. Therefore, the present invention also provides a method for producing hybrid seeds comprising:
cross-pollinating the temperature-sensitive male sterile plant according to claim 2, cultivated under a restricted temperature and made male-sterile, with a given plant; and
collecting seeds from the temperature-sensitive male sterile plant.

In the production of the hybrid seed of the present invention, the temperature-sensitive male sterile plant of the present invention is cultivated under a restricted temperature, and the plant in a male-sterile state is used.

The "restricted temperature" means a temperature above the cultivation temperature (permissive temperature) at which the temperature-sensitive male sterile plant of the present invention can form pollen. Those skilled in the art can appropriately adjust such restricted temperature and permissive temperature according to the type of plant and the like.

Further, cultivation under the restricted temperature may be carried out using a known cultivation method suitable for the type of plant and the like. In addition, the cultivation period is not particularly limited as long as it includes the period from flower bud formation (flowering) to pollen formation. The term "flower bud" means a meristem that underwent vegetative growth and has differentiated into a meristem for reproductive growth, that is, the primordium of a flower. The formation period varies depending on the type of plant, variety/strain thereof, cultivation conditions, and the like, but can be determined by those skilled in the art by a known method (for example, visual observation). In addition, cultivation under the restricted temperature during that period may be continuous (for example, cultivation under the restricted temperature during the day and at night), or may be intermittent (for example, cultivation under the restricted temperature only during the day).

The "given plant" to be crossed with the temperature-sensitive male sterile plant of the present invention is not particularly limited as long as it maintains male fertility, and examples thereof include plants that are different in lines or varieties from but the same in species with the temperature-sensitive male sterile plant of the present invention.

The "cross-pollination" between the temperature-sensitive male sterile plant of the present invention and a given plant, and the "collection of seeds" formed thereby can be appropriately carried out by those skilled in the art using a known method according to the type of plant and the like.

In addition, the seeds thus obtained are first-generation hybrid (F1) seeds, so-called hybrid seeds, with the temperature-sensitive male sterile plant of the present invention as the mother plant (maternal line), and a given plant as the father plant (paternal line). Therefore, the present invention also provides
a hybrid seed comprising: the temperature-sensitive male sterile plant of the present invention, cultivated under a restricted temperature and made male-sterile, as a mother plant; and a given plant as a father plant.

### [Examples]

The present invention is described in more detail based on Examples below, but the present invention is not limited to the following Examples. In addition, the present Examples were carried out using the materials and methods shown below, except for Example 5 relating to tomatoes, which is described later.

### (Variety and Line)

For rice, the standard variety "Nipponbare" as well as the temperature-sensitive male sterile line "Rice Norin PL 12" (PL12) and its original variety "Reimei" were used. In addition, a practical high-yield rice variety "Hokuriku 193" (Indica type rice) was also used. For Arabidopsis thaliana, the standard ecotype Columbia was used.

### (Plant Cultivation Method)

Rice was cultivated in pots under its normal cultivation conditions (28°C during the day/22°C at night). Flowering was induced under short-day conditions, and cultivation continued at 28°C during the day/22°C at night or 33°C during the day/22°C at night. The formation of pollen was observed at the time of heading, and seed fertility was confirmed.

Arabidopsis thaliana was cultivated in a growth chamber set to 21°C continuous light conditions. After bolting, cultivation was continued at 21°C or 27°C, and pollen formation and fruiting were observed.

### (Genome Editing of Rice)

The CRISPR/Cas9 method was used, which is commonly used for plants. More specifically, a guide RNA, Cas9, and a vector expressing a hygromycin-resistant gene as a selection marker were introduced into callus of rice variety "Nipponbare" or immature embryos of "Hokuriku 193" by the Agrobacterium method. Note that the vector is based on pPZP202 and can express guide RNA and Cas9 under the control of the rice U6 promoter and the rice ubiquitin promoter, respectively. Next, re-differentiated plants were obtained from the callus selected for hygromycin resistance, genomic DNA was prepared from their leaves, and the nucleotide sequence of the genome editing portion was confirmed. Mutations such as single-base insertions that deviate from the reading frame of codons were selected. These mutants are gene-deficient because they cannot express the protein for which this gene functions.

Then, the rice variety "PL12" as well as " Nipponbare," "Hokuriku 193," and genome-edited lines thereof were cultivated in pots in a greenhouse under normal cultivation conditions (28°C during the day/22°C at night). Flowering was induced under short-day conditions, and cultivation continued at 28°C during the day/22°C at night or high temperature conditions (33°C during the day/22°C at night for " Nipponbare" and 35°C during the day/25°C at night for "Hokuriku 193"), and pollen formation was observed at the time of heading.

### (Genome Editing of Arabidopsis thaliana)

As with rice described above, genome editing was carried out using the CRISPR/Cas9 method. The method of gene introduction is different from that of rice described above. For the transformation of Arabidopsis thaliana, the commonly used floral dip method was used.

A temperature-sensitive male sterile line "Rice Norin PL 12" was selected for cultivation from a gamma-irradiated mutant population of the variety "Reimei". This strain normally forms pollen and bears fruit at normal cultivation temperatures (about 28°C), but at high temperatures (about 33°C) pollen formation is impaired and no fruiting is observed (see Fig. 3). In addition, no abnormalities were observed in vegetative organs such as leaves and pistils, and it is known that the susceptible period is several days around 20 days before heading. Genetic analysis has shown that this temperature-sensitive male sterility trait is due to a recessive single factor, and it has been reported that the responsible mutation is located on chromosome 7 (see NPL 1). Therefore, an attempt was made to identify the mutation responsible for the temperature-sensitive male sterility trait as follows.

### (Example 1)

Fine mapping was performed to identify the mutation responsible for the temperature-sensitive male sterility trait, but could not narrow the range down further from about 1.8 Mb near 54 cM. Therefore, whole genome sequence analysis was performed on the original variety "Reimei" and "PL12," and the two were compared. As a result, it was found that there was a deletion of about 150 kb in the range of about 1.8 Mb of "PL12."

Next, by the CRISPR/Cas9 method using two guide RNAs, a series of partial deletions was created by introducing double-strand breaks at two locations in the genomic DNA and deleting the DNA fragment between them. More specifically, as shown in Fig. 4, the deletion region of about 150 kb was divided into three approximately equal parts to create lines having ranges with partial deletion, (1) to (2), (2) to (3), and (3) to (4). Note that to delete (1) to (2), (2) to (3), and (3) to (4), SEQ ID NOs: 23 and 24, 24 and 25, and 25 and 26 were used as target sequences to create two double-strand breaks by CRISPR/Cas9 in each of them.

As a result, as shown in Fig. 5, one of these (#571) was found to exhibit the same phenotype as "PL12," and the candidate region was narrowed down to the range of (3) to (4).

Similarly, three partial deletions were designed inside the deletion region #571. More specifically, as shown in Fig. 4, the region (3) to (4) was divided into approximately three to create lines having with partial deletion, (3) to (5), (5) to (6), and (6) to (4). Note that to delete (3) to (5), (5) to (6), and (6) to (4), SEQ ID NOs: 25 and 27, 27 and 28, and 28 and 26 were used as target sequences to create two double-strand breaks by CRISPR/Cas9 in each of them.

As a result, as shown in Fig. 5, #592 exhibited the trait of temperature-sensitive male sterility, suggesting that the responsible mutation lied in the range of approximately 10 kb from (3) to (5). From the information in the database, it was predicted that there were two genes (ORF1 and ORF2 shown in Fig. 4) in this region.

Therefore, a guide RNA was designed in each coding region, and an attempt was made to disrupt the gene function by introducing a frameshift mutation. Note that the amino acid sequence encoded by ORF1 is set forth in SEQ ID NO: 1, and the amino acid sequence encoded by ORF2 is set forth in SEQ ID NO: 29. The target sequence in the guide RNA for ORF1 is set forth in SEQ ID NO: 30 or 31. The target sequence in the guide RNA for ORF2 is set forth in SEQ ID NO: 32. In addition, Table 2 shows examples of mutations obtained for ORF1.

**[Table 2]**

| Mutation in DNA Sequence | Amino Acid Sequence Presumed |
|---|---|
| Insertion of One Base A | Same as wild type up to 52nd K, ERQICGHV* from 53rd |
| Insertion of Two Bases AA | Same as wild type up to 52nd K, RASNLR... from 53rd |
| Deletion of Two Bases AA | Same as wild type up to 52nd K, RQICGHV* from 53rd |

As a result, as shown in Fig. 5, line #612 exhibited the trait of temperature-sensitive male sterility. Furthermore, although not shown in the figure, not limited to representative examples of mutant strains shown in Fig. 5, it was confirmed that a frameshift mutation led to the expression of the trait of temperature-sensitive male sterility, and it was found that the loss of gene function in ORF1 was the mutation responsible for "PL12."

This gene is predicted as a gene with an ID of LOC_Os07g26794 in the MSU database and an ID of Os07g0482700 in the RAP-DB, but it is a novel gene whose function is unknown. This gene is widely conserved in living organisms, with a high conservation especially in plant species. A comparison of predicted amino acid sequences in higher plants is shown in Fig. 6. Sequence conservation is even higher among rice crops (see Fig. 7). In addition, the temperature-sensitive male sterility gene thus newly identified was named TMS2.

### (Example 2)

The genome-edited lines lacking the gene were cultivated at the permissive temperature and restricted temperature in comparison with the standard variety " Nipponbare" and the temperature-sensitive male sterile line "PL12" before genome editing, and the appearance of the flower was observed. As a result, as shown in Fig. 8, pollen was formed normally in the standard variety "Nipponbare" regardless of the temperature. Meanwhile, the temperature-sensitive male sterile line "PL12" normally formed pollen at 28°C and bore yellow and full anthers, but at 33°C did not develop pollen and bore anthers with abnormal morphology and color. Then, the line lacking the gene in "Nipponbare" by genome editing exhibited the phenotype of temperature-sensitive male sterility as in "PL12."

Furthermore, the line of rice variety "Nipponbare" whose gene function had been deleted by genome editing was cultivated at 28°C and 33°C. As a result, as shown in Fig. 9, at 28°C, the fruit was normally borne and the ears were drooping, whereas at 33°C, no fruit was borne and the ears were standing.

### (Example 3)

Genome editing was carried out in the same manner as in Example 2 ("Nipponbare" (Japonica type rice)) using a practical high-yield rice variety "Hokuriku 193" (Indica type rice), and the temperature-sensitive male sterility gene was deleted. Note that the transformation of "Hokuriku 193" was performed with reference to Yukoh Hiei, "Research on rice transformation methods using Agrobacterium tumefaciens," 2014 Nagoya University dissertation. Then, the genome-edited line derived from "Hokuriku 193" thus obtained was cultivated in a greenhouse set to normal conditions (28°C) or high temperature conditions (35°C), and the formed anthers were observed.

As a result, as shown in Fig. 10, it was confirmed that as with the genome-edited line derived from "Nipponbare," the genome-edited line derived from "Hokuriku 193" also formed pollen and developed anthers under normal conditions, but became male sterile under high-temperature conditions due to imperfect pollen formation.

In addition, at the flowering stage, pollen of another high-yielding variety "Oonari"' was applied to the genome-edited line derived from "Hokuriku 193," and the fertile seeds were allowed to germinate for cultivation. Then, the present inventors purified genomic DNA from the leaves of each of (1) "Oonari," (2) the genome-edited line derived from "Hokuriku 193," and (3) the individuals obtained by applying the pollen of "Oonari" to the genome-edited line derived from "Hokuriku 193" cultivated at high temperature, and analyzed the base sequence of the genome-edited site (mutation insertion site) of the temperature-sensitive male sterility gene.

As a result, as shown in Fig. 11, the individual (3) was a heterozygote of the genomic sequences of (1) and (2), indicating that crossing of (1) and (2) resulted in a hybrid. Therefore, it was confirmed that pistils functioned normally even when the genome-edited line was cultivated at high temperature, and that the genome-edited line could actually be put into use for crossing.

### (Example 4)

For the dicotyledonous plant Arabidopsis thaliana as well, an attempt was made to create a temperature-sensitive male sterile line by deleting the gene. More specifically, genome editing was performed by the CRISPR/Cas9 method to create a line in which the gene had ceased to function.

Note that the target sequence in Arabidopsis thaliana is set forth in SEQ ID NO: 33. In addition, Table 3 shows examples of the obtained mutations.

**[Table 3]**

| Mutation in DNA Sequence | Amino Acid Sequence Presumed |
|---|---|
| Insertion of One Base A | Same as wild type up to 18th K, EARGFVRV* from 19th |
| Deletion of One Base A | Same as wild type up to 17th I, RGKRFR* from 18th |

Then, the lines of Arabidopsis thaliana whose gene function had been deleted by genome editing were cultivated at 21°C and 27°C. As a result, as shown in Fig. 12, at 21°C, the fruit was normally borne and the pods were the same in length as the wild type, but at 27°C, no fruit was borne and the pods did not elongate.

Note that although not shown in the figure, it was confirmed that Arabidopsis thaliana, as well as the representative examples of the mutant strains shown in Fig. 12, exhibited the above-mentioned trait related to temperature-sensitive male sterility if frameshift mutation occurs, as in the case of rice described above.

In addition, in the same manner as in Example 3, a crossing experiment was also conducted for Arabidopsis thaliana. As a result, although not shown in the figure, seeds were obtained. Therefore, it is suggested that the genome-edited line can be used for crossing in Arabidopsis thaliana, without affecting the functions of the pistil, as in the case of rice described above.

### (Example 5)

Genome editing of temperature-sensitive male sterility gene was performed on tomato to establish mutant lines. Specifically, first, the genome sequence information of the tomato temperature-sensitive male sterility gene was obtained from Ensembl Plants. Note that the cDNA (wild-type) sequence of the gene is set forth in SEQ ID NO: 43. Also, the amino acid sequence of the protein encoded by the cDNA is set forth in SEQ ID NO: 9 or 44 (SEQ ID NOs: 9 and 44 show the same amino acid sequence).

Then, targeting the obtained genome sequence information, CRISPR/Cas target sequences were searched on CRISPRdirect (http://crispr.dbcls.jp/) . As a result, the following two sequences, located within exons and having no similar sequences on the tomato genome, were selected as genome editing target sequences.
GE51 (within exon 3)
5'-ACCATAGGTGAGAAGTCACGAGG-3' (SEQ ID NO: 37)
GE52 (within exon 4)
5'-CCAGGCTGTCTACCAGAGAAATG-3' (SEQ ID NO: 38).

Next, a genome editing vector was prepared. A genome editing vector for plant genome editing, pEgP237-2A-GFP, was used as the vector. pEgP237-2A-GFP was provided by Professor Osakabe at Tokushima University. For the details of the vector, see Ueta et al. (2017) Rapid breeding of parthenocarpic tomato plants using CRISPR/Cas9. Sci Rep. 7: 507.

pEgP237-2A-GFP was digested with BsaI and then purified. Next, after mixing equal amounts of the following chemically synthesized oligo DNAs, the mixture was denatured by heat and annealed. The resulting double-stranded oligo DNA was mixed with BsaI-digested pEgP237-2A-GFP, inserted into the vector, and ligated. Then, it was introduced into Escherichia coli to amplify the genome editing vector.
#51
   Sl_tms2_gRNA01F
   5'-GATTGACCATAGGTGAGAAGTCACG-3' (SEQ ID NO: 39)
   Sl_tms2_gRNA01R
   5'-AAACCGTGACTTCTCACGATACCA-3' (SEQ ID NO: 40)
#52
   Sl_tms2_gRNA02F
   5'-GATTGCATTTCTCTGGTAGACAGCG-3' (SEQ ID NO: 41)
   Sl_tms2_gRNA02R 5'-AAACGGCTGTCTACCAGAGAAATG-3' (SEQ ID NO: 42).

Then, the genome editing vector thus constructed was transformed into Rhizobium radiobacter (Agrobacterium tumefaciens) GV2260, and further transformation of tomato variety "Microtom" was performed using the Agrobacterium method. Note that for tomato transformation methods, see Sun et al. (2006) A Highly Efficient Transformation Protocol for MicroTom, a Model Cultivar for Tomato Functional Genomics. Plant Cell Physiol. 47: 426-431.

The target sequences of the transformants thus obtained were confirmed, and genome-edited individuals were obtained. In the current transgenic generation, no individual homozygous for the mutation was obtained. Therefore, seeds were obtained by self-breeding, and individuals were selected in which the mutation was fixed from the progeny.

Next, seedlings of the obtained genome-edited individuals were grown at room temperature of 23°C/18°C (day/night) for a day length of 14 hours until flowering. Further, the they were transferred to a high temperature of 30°C/28°C (day/night) for a day length of 14 hours, and the first flowers were removed. After that, the presence or absence of seeds in the fruits borne was confirmed. Fig. 13 shows the obtained results.

As shown in Fig. 13, for tomato as well, seeds were fruited under normal conditions due to the deletion of the temperature-sensitive male sterility gene, whereas no seeds were formed under high-temperature conditions, and a temperature-sensitive phenotype was observed as in rice. Tomato, as with rice, is a self-pollinated plant, and its pollen adheres to the pistils to form seeds. This indicates the applicability to crops such as vegetables.

Finally, Fig. 14 summarizes the amino acid sequences encoded by the temperature-sensitive male sterility genes derived from various plants used in the present Examples and the mutation sites found in the genome-edited individuals prepared.

### [Industrial Applicability]

As described above, the present invention makes it possible to produce a temperature-sensitive sterile plant. In particular, temperature-sensitive sterile plants can be produced without requiring special breeding other than suppressing the function of the temperature-sensitive male sterility gene of the present invention.

Moreover, as shown in Figs. 6 and 7, the amino acid sequences of the proteins encoded by the temperature-sensitive male sterility gene of the present invention are highly conserved. Therefore, in the method of the present invention, the lines to be used are not limited, so that it is possible to produce male sterile lines by suppressing the function of this gene in myriad varieties and lines of myriad plant species. For example, simply suppressing the function of a temperature-sensitive male sterility gene in a useful variety can be used immediately for hybrid production.

Then, by cultivating the obtained temperature-sensitive male sterile plant as a mother plant together with another line as a father plant under a restricted temperature, it is possible to easily obtain hybrid seeds in which these two lines are crossed.

Thus, the present invention is expected to have a great impact on agricultural production, and is a very useful technique in this field.

## Claims

1. A method for producing a temperature-sensitive male sterile plant, comprising:
artificially suppressing a function of at least one gene selected from the group consisting of (a) to (d) below
(a) a gene encoding a protein composed of an amino acid sequence set forth in any one of SEQ ID NOs: 1 to 22,
(b) a gene encoding a protein composed of an amino acid sequence in which one or more amino acids are substituted, deleted, added, and/or inserted in the amino acid sequence set forth in any one of SEQ ID NOs: 1 to 22,
(c) a gene encoding an amino acid sequence having 60% or more homology with the amino acid sequence set forth in any one of SEQ ID NOs: 1 to 22, and
(d) a gene containing a DNA that hybridizes under stringent conditions with a DNA composed of a nucleotide sequence encoding the amino acid sequence set forth in any one of SEQ ID NOs: 1 to 22.

2. A temperature-sensitive male sterile plant with artificially suppressed functions of at least one gene selected from the group consisting of (a) to (d) below
(a) a gene encoding a protein composed of an amino acid sequence set forth in any one of SEQ ID NOs: 1 to 22,
(b) a gene encoding a protein composed of an amino acid sequence in which one or more amino acids are substituted, deleted, added, and/or inserted in the amino acid sequence set forth in any one of SEQ ID NOs: 1 to 22,
(c) a gene encoding an amino acid sequence having 60% or more homology with the amino acid sequence set forth in any one of SEQ ID NOs: 1 to 22, and
(d) a gene containing a DNA that hybridizes under stringent conditions with a DNA composed of a nucleotide sequence encoding the amino acid sequence set forth in any one of SEQ ID NOs: 1 to 22.

3. A method for producing hybrid seeds comprising:
cross-pollinating the temperature-sensitive male sterile plant according to claim 2, cultivated under a restricted temperature and made male-sterile, with a given plant; and
collecting seeds from the temperature-sensitive male sterile plant.

4. A hybrid seed comprising:
the temperature-sensitive male sterile plant according to claim 2, cultivated under a restricted temperature and made male-sterile, as a mother plant; and
a given plant as a father plant.
